# EUROPEAN PATENT APPLICATION

(11) **EP 4 789 643 A1**
(43) Date of publication of application: **12.08.2026**
(21) Application number: 26156437.1
(22) Date of filing: 04.02.2026
(51) Int. Cl.: A61F 5/01

(54) **ARTICULATED ROD ADJUSTABLE IN FLEXION-EXTENSION**

(30) Priority: 05.02.2025 IT 202500002184
(71) Applicant: Orthoservice AG, 6830 Chiasso (CH)
(72) Inventor: TURCONI, Francesco, 20900 Monza (MB) (IT); RAVESE, Mauro, 20852 Villasanta (MB) (IT); GALLINA, Simone, 31010 Maser (TV) (IT)
(74) Representative: Branca, Emanuela

(57) **Abstract**

An articulated rod adjustable in flexion-extension (10) comprises a pair of arms (20), a pair of outer and inner cover plates (31, 32), and two rotation pins (24) engaged in through holes (23) of the pair of arms (20) and in through holes (33, 34) of the pair of cover plates (31, 32), to form a polycentric joint (11) articulating the pair of arms (20) in mutual rotation, wherein the rotation comprises a flexion rotation direction and an extension rotation direction, as well as two adjusting pins (27) integral with the arms (20), wherein the articulated rod (10) comprises a pair of discs (40), provided on at least a perimeter portion with a plurality of interlocking teeth (42) and a position indicating cursor (43), and comprises a locking element (50) adapted to engage with the plurality of interlocking teeth (42) of the pair of discs (40) and with at least the outer cover plate (31), and preferably also with the inner cover plate (32), wherein the discs (40) are axially hinged with the rotation pins (24) and are arranged partially overlapping on parallel rotation planes, wherein each disc (40) bears a slot (44) shaped as an arc of a circle, which limits the movement of the adjusting pin (27) integral with each arm (20).

## Description

The present invention relates to an articulated rod adjustable in flexion-extension, in particular for an orthopedic device such as a knee brace or an elbow brace.

In the state of the art, there are articulated rods of orthopedic devices, in particular articulated rods with a polycentric joint provided with removable inserts to constrain a flexion-extension rotation within a determined range. In these articulated rods, it is necessary to use inserts of different shapes and sizes to constrain different ranges of rotation.

Disadvantageously, whenever it is desired to modify the rotation range, it becomes necessary to dismount the inserts from the joint and replace them with others, making the adjustment operation complicated as being related to operations of mounting and dismounting the inserts.

Indeed, it is necessary to have tools for applying and removing the inserts, as well as to store the various inserts provided with each product in order to perform different subsequent adjustments.

The operation of applying and removing the inserts further requires good manual skills by the operator.

The Applicant has already tested an articulated rod for orthopedic devices, which uses a different adjustment system of the flexion-extension rotation range compared to the removable inserts, for example as shown in EP 3492051 A1.

The main drawback of this solution is its criticality in terms of mechanical strength when subjected to load.

The object of the present invention is to make an articulated rod adjustable in flexion-extension that is easy to adjust and has adequate mechanical strength under load.

Another object of the present invention is to provide an articulated rod adjustable in flexion-extension that is particularly simple and functional, with contained costs.

These objects according to the present invention are achieved by making an articulated rod adjustable in flexion-extension as set forth in claim 1.

Further features are provided in the dependent claims.

The features and advantages of an articulated rod adjustable in flexion-extension according to the present invention will become more apparent from the following exemplary and non-limiting description, referred to the attached schematic drawings in which:

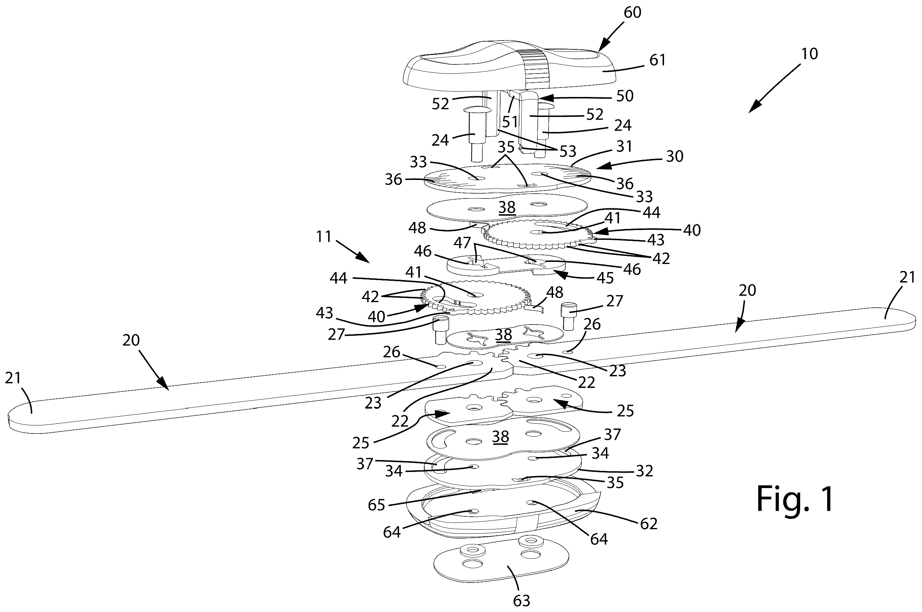

Figure 1 is an exploded perspective view of an articulated rod adjustable in flexion-extension according to the invention;

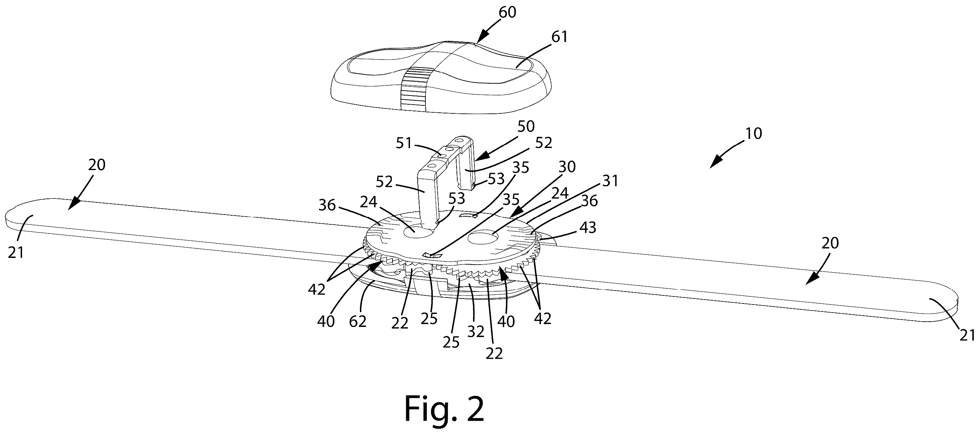

Figure 2 shows the rod of Figure 1 with only the cover and the locking element removed;
Figure 3 is a plan view of the articulated rod according to the invention, shown with some components removed for illustrative purposes;
Figures 4A, 4B, and 4C exemplarily show a sequence of three possible adjustment configurations of the articulated rod according to the invention, shown schematically in different angular locking positions, wherein the flexion-extension is prevented;
Figures 5A, 5B, and 5C exemplarily show a sequence of three possible adjustment configurations of the articulated rod according to the invention, shown schematically in different angular positions, wherein the flexion-extension is allowed from a maximum possible extension position corresponding to the rod being straight (0°) to a variable maximum possible flexion position (from 30° to 120°);
Figures 6A, 6B, and 6C exemplarily show a sequence of three possible adjustment configurations of the articulated rod according to the invention, shown schematically in different angular positions, wherein the flexion-extension is allowed from a maximum possible extension position lower than the straight rod condition and variable (from 45° to 105°) to a variable maximum possible flexion position (from 60° to 135°).

With reference to the figures, an articulated rod adjustable in flexion-extension, overall indicated by 10, is shown, comprising a pair of arms 20, articulated to each other in a rotation plane, and a pair of cover plates 30, an outer cover plate 31 and an inner cover plate 32, where the terms "outer" and "inner" refer to the mounting directions with respect to the orthopedic device.

Each of the arms 20 bears a first end 21 associable with an orthopedic device, such as a knee brace or an elbow brace, for example by insertion into a pocket, and an opposite toothed end 22. The toothed ends 22 of the two arms 20 are mutually engaged to perform a flexion-extension movement in a geometric rotation plane.

Each of the toothed ends 22 bears a through hole 23, placed axially with respect to the extension of the toothed profile, within which a rotation pin 24 is engaged, arranged orthogonally with respect to the rotation plane.

Each of the outer and inner cover plates, respectively 31 and 32, has a pair of through holes 33, 34, in which the rotation pins 24 are engaged, forming a polycentric joint 11, which allows mutual rotation of the pair of arms 20. The rotation comprises two rotation directions, which are a flexion rotation direction and an extension rotation direction.

In the depicted example, a pair of reinforcing gears 25 is coupled on the inner side of the toothed ends 22 of the arms 20. The pair of reinforcing gears 25 has the same toothed profile as the toothed ends 22, with which it is integrally placed in rotation. This allows for a better distribution of loads with the same cross-section of the arms 20.

In the depicted example, the rotation pins 24 are advantageously made as double-diameter rivets.

The articulated rod 10 further comprises a pair of discs 40, a locking element 50, and a cover 60.

Each disc 40 axially bears a through hole 41 in which the rotation pin 24 is engaged.

According to the invention, the discs 40 have a radius greater than half the distance between the rotation pins 24 and are therefore arranged partially overlapping on parallel rotation planes.

Each disc 40 is provided on the perimeter with a plurality of interlocking teeth 42 and a position indicating cursor 43. The interlocking teeth 42 are preferably distributed substantially over the entire perimeter, except for the provision of the indicating cursor 43, although they could concern only certain portions thereof.

Each disc 40 bears a slot 44 shaped as an arc of a circle, with an angle less than 90° and preferably greater than 60°, for example around 70°.

A spacer element 45, provided with a through hole 46 in which the rotation pin 24 is engaged, holds the respective disc 40 in position in its rotation plane.

According to a preferred embodiment of the invention, the spacer element 45 comprises, on opposite sides, a seat 47 respectively for a leaf spring 48 which works with the plurality of interlocking teeth 42 of the respective disc 40 to achieve a stepwise rotation adjustment. According to what is shown, the seat 47 of the leaf spring 48 is integrated with the through hole 46 that accommodates the rotation pin 24.

According to the preferred embodiment, the two spacer elements 45 are made as a single piece.

According to the invention, each of the arms 20 bears a second hole 26 aligned with the first hole 23 on the side opposite to the toothed part. An adjusting pin 27 is associated with the second through hole 26 of the arm 20 and, when the rod is mounted, it engages in the slot 44 of the disc 40, which limits its movement between two endstroke positions.

The locking element 50 comprises two interlocking appendages 52, each one adapted to engage with the plurality of interlocking teeth 42 of both discs 40 on two opposite sides and with at least the outer cover plate 31, and preferably also with the inner cover plate 32.

According to the preferred embodiment shown, the locking element 50 also comprises a crosspiece 51 from which the two interlocking appendages 52 extend, forming a "bridge." The interlocking appendages 52 can also be directly constrained to the cover 60.

According to a preferred embodiment, the outer cover plate 31, and preferably also the inner cover plate 32, bear a pair of through holes 35 shaped in a complementary manner with the interlocking appendages 52, in order to stably accommodate the locking element 50 in the working position. In the example, the interlocking appendages 52 bear, at one end, a toothed portion 53.

The outer cover plate 31 preferably bears respective graduated scales 36 for setting the indicating cursor 43 to the desired flexion-extension angles.

According to a preferred embodiment, the adjusting pins 27 are connected in a passing way to the pair of arms 20, and the inner cover plate 32 comprises a pair of slots 37 with an arc-of-a-circle profile comparable to the slots of the discs 40, which accommodate in rotation the lower end of the adjusting pin 27. This solution provides a further stabilizing effect and prevents possible friction between the adjusting pin 27 and the inner cover plate 32. Furthermore, advantageously, the adjusting pin 27 is also constrained to the reinforcing gears 25, contributing to distribute the load acting on the adjusting pin 27 during operation. The adjusting pins 27 are, for example, of the rivet type.

The cover 60 is divided into two plastic-material half-shells that can be coupled to each other and to the arms 20. An outer half-shell 61 can be made integral with the locking element 50 to achieve the removal of the locking element 50 by removing the cover to perform adjustments, or it can have only a covering function. An inner half-shell 62 can be provided with a gripping means 63, for example Velcro^{®}, to stabilize its positioning on the orthopedic device.

Furthermore, the inner half-shell 62 can be directly constrained to the rotation pins 24 passing through the pair of holes 64.

The inner cover 62 is preferably provided with a pair of holes 65 for snap engagement with the toothed portion 53 of the interlocking appendages 52 of the locking element 50.

The articulated rod 10 according to the invention comprises sealing elements 38, made in the form of polymeric-material sheets, interposed between the components placed in respective rotation both during the flexion-extension movement and during the adjustment step.

Advantageously, in order to increase the strength under load without compromising the weight, only the most stressed components are made of steel, such as the cover plates 30, the reinforcing gears 25, the first and second pins, 24 and 27, the discs 40, and the locking element 50.

The pair of arms 20 is preferably made of aluminium; the sealing elements 38, the cover 60, and the spacer elements 45 are made of polymeric material.

The articulated rod 10 limits the flexion-extension of the limb according to a range of flexion angles α that can be predetermined based on the adjustment set, the so-called "range of motion" (ROM), which can vary, for example, in a healthy limb from approximately 0° to 135°, that is, from a maximum extension angle α_{E} equal to 0° to a maximum flexion angle α_{F} equal to 135°.

Therefore, setting the ROM of the articulated rod 10, for example between α_{E} = 30° and α_{F} = 90°, means limiting the range of the articulation flexion-extension movement between flexion angles α between 30° and 90° with respect to the fully extended articulation position, corresponding to the arms 20 being aligned, thus preventing both a full extension of the articulation and a flexion beyond 90°.

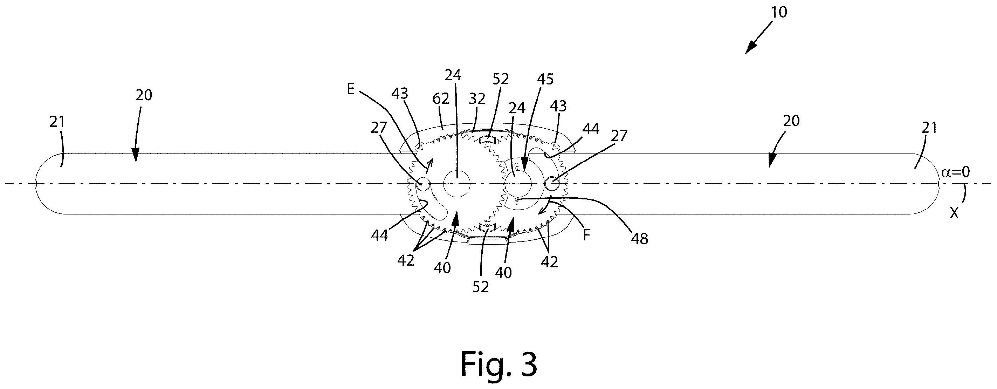

In the figures, for example in Figure 3, the flexion angles α are depicted as symmetrically divided between the two arms 20 as angles α/2 with respect to the maximum extension position, with the arms 20 aligned, indicated by an axis X.

Figure 3, which shows the articulated rod 10 in a plan view with some components removed, such as the outer cover 61, the crosspiece 51 of the locking element 50, and the outer cover plate 31, is exemplarily set with the maximum extension angle α_{E} equal to 0 and a maximum flexion angle α_{F} equal to 0. The articulated rod 10 is thus adjusted in a fixed straight position, with no movement possibility. Indeed, each of the adjusting pins 27 is in abutment at one end of the slot 44, thereby preventing both flexion rotation and extension rotation of the pair of arms 20, respectively schematized by arrows F and E in figure. The two orthogonal appendages 52 of the locking element 50 both engage a short section of the perimeter of the discs 40, in the example, two teeth 42 of both discs 40, ensuring that they cannot rotate and therefore the set maximum extension α_{F} and flexion α_{F} angles cannot be modified.

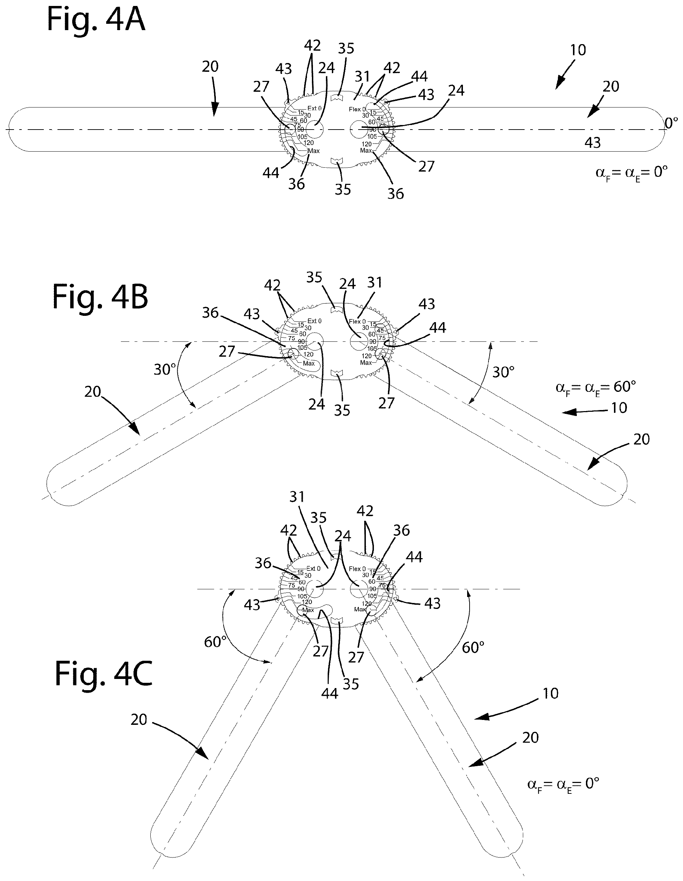

In Figures 4A, 4B, and 4C, three possible adjustment configurations of the articulated rod 10 according to the invention are schematically shown for exemplary purposes in sequence, in different angular locking positions, in which the flexion-extension rotation is prevented. This condition is achieved by setting the same values for both the maximum flexion angle α_{F} and the maximum extension angle α_{E}, in the examples respectively 0°, 60°, and 120°. The slots 44 of the discs 40 are schematically shown as solid lines, even though hidden by the outer cover plate 31, in order to better show the positioning of the adjusting pins 27 with respect to the slots 44 in the various angular configurations.

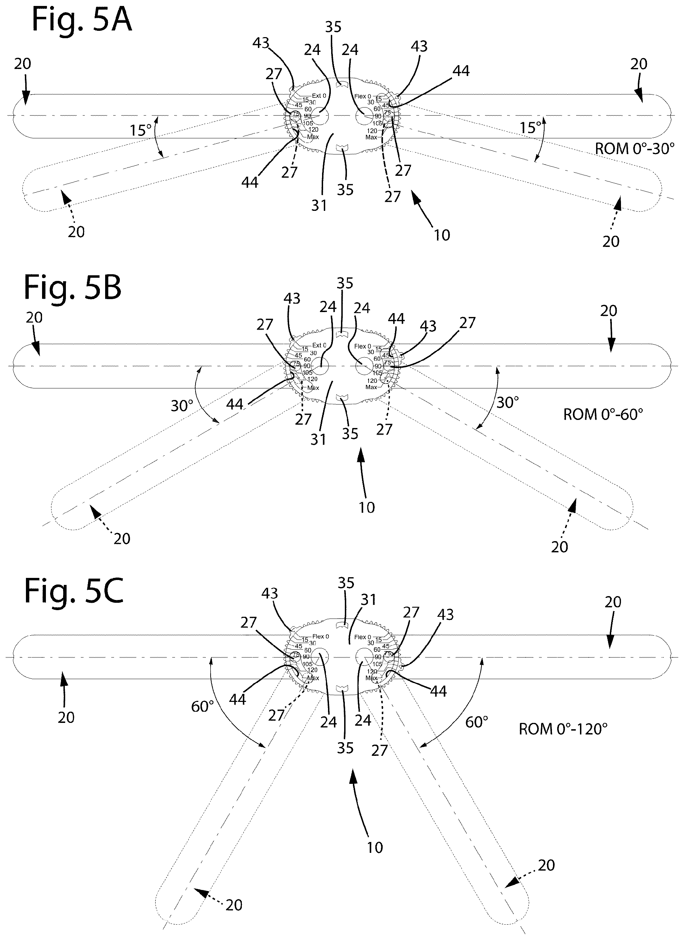

In Figures 5A, 5B, and 5C, three possible adjustment configurations of the articulated rod 10 according to the invention are schematically shown for exemplary purposes in sequence, with different ROM settings for the flexion-extension rotation. All the shown configurations start from a maximum possible extension position corresponding to the straight rod condition, with α_{F} equal to 0°, shown as a solid line, up to a maximum possible flexion position with α_{F} varying from 30° to 120°, in which the arms 20 and adjusting pins 27 are shown in dashed lines. The slots 44 of the discs 40 are schematically shown as solid lines, even though hidden by the outer cover plate 31, to better show the positioning of the adjusting pins 27 with respect to the slots 44 in the different angular configurations at both the maximum and minimum flexion-extension positions of the ROM.

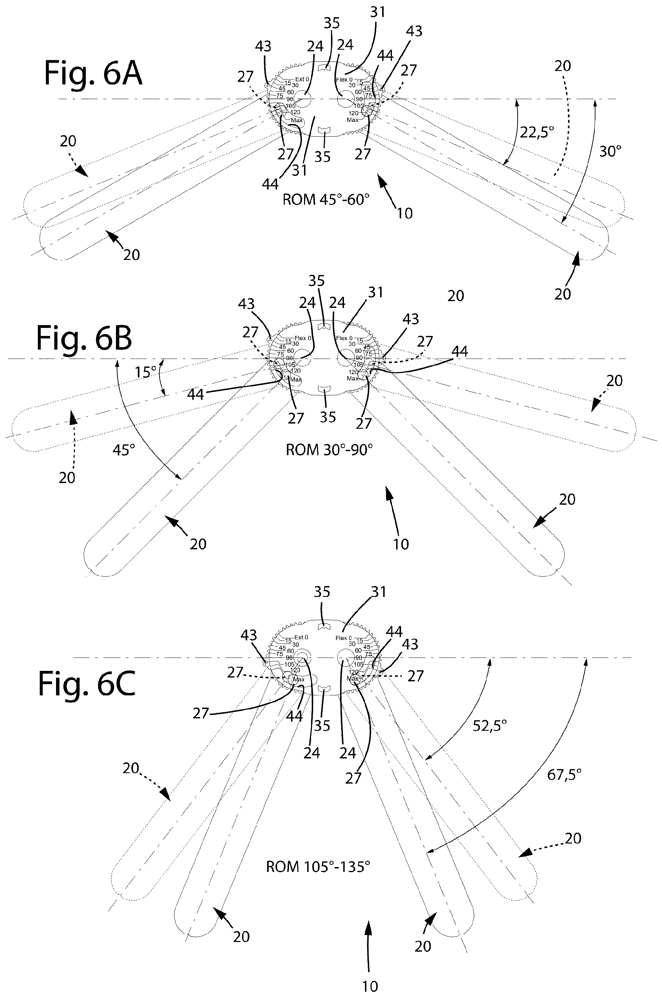

In Figures 6A, 6B, and 6C, three possible adjustment configurations of the articulated rod 10 according to the invention are schematically shown for exemplary purposes in sequence, with different ROM values, in which the flexion-extension is allowed from a maximum possible extension position, lower than the straight rod condition, wherein the arms 20 and adjusting pins 27 are shown in dashed lines, to a maximum possible flexion position that varies among the different configurations, shown as a solid line. In the shown configurations, the ROM varies respectively from 45° to 60°, from 30° to 90°, and from 105° to 135°. Therefore, both the range of the permitted flexion-extension and the respective maximum extension and flexion positions defined by the angles α_{E} and α_{F} vary. The slots 44 of the discs 40 are schematically shown as solid lines, even though hidden by the outer cover plate 31, in order to better show the positioning of the adjusting pins 27 with respect to the slots 44 in the different angular configurations, in both the maximum and minimum flexion-extension positions of the ROM.

The articulated rod adjustable in flexion-extension according to the present invention has the advantage of good mechanical resistance under load.

Advantageously, in the articulated rod according to the invention, the locking element engages only a few teeth, and therefore less precision is required in the making of the components. Furthermore, the insertion of the locking element when setting the desired adjustment is also facilitated.

The toothed discs are advantageously made of steel and have a relatively large diameter.

Making the slot in a position separated from the through holes for the rotation pins contributes to increased mechanical strength.

The articulated rod adjustable in flexion-extension thus conceived is susceptible to numerous modifications and variants, all falling within the invention; furthermore, all details are replaceable by technically equivalent elements. In practice, the materials used, as well as the dimensions, can be any depending on the technical requirements.

## Claims

1. Articulated rod (10) adjustable in flexion-extension comprising a pair of arms (20), a pair of outer and inner cover plates (31, 32) and two rotation pins (24), engaged in through holes (23) of the pair of arms (20) and in through holes (33, 34) of the pair of cover plates (31, 32) to form a polycentric joint (11) articulating the pair of arms (20) in mutual rotation, wherein the rotation comprises a flexion rotation direction and an extension rotation direction, as well as two adjusting pins (27) integral with the arms (20), wherein the articulated rod (10) comprises a pair of discs (40), provided on at least a perimeter portion with a plurality of interlocking teeth (42) and a position indicating cursor (43), and comprises a locking element (50) adapted to engage with the plurality of interlocking teeth (42) of the pair of discs (40) and with at least the outer cover plate (31) and preferably also with the inner cover plate (32), wherein the discs (40) are axially hinged with the rotation pins (24) and are arranged partially overlapping on parallel rotation planes, wherein each disc bears a slot (44) shaped as an arc of a circle, which limits the movement of the adjusting pin (27) integral with each arm (20).

2. Articulated rod according to claim 1, **characterised in that** the slot (44) of each disc (40) describes a circular arc with an angle less than 90° and preferably greater than 60°.

3. Articulated rod according to claim 1 or 2, **characterised in that** it comprises for each disc (40) a spacer element (45) provided with a through hole (46) for the rotation pin (24), which holds the respective disc (40) in position in its rotation plane.

4. Articulated rod according to claim 3, **characterised in that** the spacer element (45) comprises two seats (47) for respective leaf springs (48) which work with the plurality of interlocking teeth (42) of the respective disc (40) to achieve a stepwise rotation adjustment.

5. Articulated rod according to claim 1, **characterised in that** the locking element (50) comprises two interlocking appendages (52), each one adapted to engage with the plurality of interlocking teeth of both discs (40) on two opposite sides and preferably provided at the ends with a toothed portion (53)

6. Articulated rod according to any one of the preceding claims, **characterised in that** at least the outer cover plate (31) bears a pair of through holes (35) shaped in a complementary manner with the interlocking appendages (52) to stably accommodate the locking element (50) in a working position.

7. Articulated rod according to any one of the preceding claims, **characterised in that** the outer plate (31) bears respective graduated scales (36) for setting the indicating cursor (43) of the discs (40) to the desired flexion-extension angles.

8. Articulated rod according to any one of the preceding claims, **characterised in that** each adjusting pin (27) is connected in a passing way to the arm (20) and the inner cover plate (32) comprises a pair of slots (37) with the same arc-of-a-circle profile of the slots (44) of the discs (40), which rotatably accommodate the lower end of the adjusting pin (27).

9. Articulated rod according to any one of the preceding claims, **characterised in that** it comprises a cover (60) divided into two outer and inner half-shells (61, 62) which can be coupled to each other and to the arms (20), wherein the inner half-shell (62) is preferably constrained with the rotation pins (24) and the locking element (50).

10. Articulated rod according to any one of the preceding claims, **characterised in that** it comprises a pair of reinforcing gears (25) having the same toothed profile as the toothed ends (22) of the arms (20) and coupled to the toothed ends (22) by means of the rotation pins (24) and the adjusting pins (27).
